(19) Europäisches Patentamt
European Patent Office
Office européen des brevets

(11) **EP 3 841 964 A1**

(12) **DEMANDE DE BREVET EUROPEEN**

(43) Date de publication:
**30.06.2021 Bulletin 2021/26**

(51) Int Cl.:
*A61B 5/00* (2006.01)          *A61B 5/16* (2006.01)
*G06N 7/02* (2006.01)

(21) Numéro de dépôt: **20217202.9**

(22) Date de dépôt: **24.12.2020**

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**
Etats d'extension désignés:
**BA ME**
**KH MA MD TN**

(30) Priorité: **26.12.2019 FR 1915596**

(71) Demandeurs:
• **Commissariat à l'énergie atomique et aux énergies alternatives**
  **75015 Paris (FR)**

• **Université Grenoble Alpes**
  **38400 Saint-Martin-d'Hères (FR)**

(72) Inventeurs:
• **VILA, Gaël**
  **38054 GRENOBLE CEDEX 09 (FR)**
• **GODIN, Christelle**
  **38054 GRENOBLE CEDEX 09 (FR)**

(74) Mandataire: **INNOV-GROUP**
  **310, avenue Berthelot**
  **69372 Lyon Cedex 08 (FR)**

(54) **PROCÉDÉ DE DÉTERMINATION D'UNE FONCTION D'APPARTENANCE, DESTINÉE À ÊTRE APPLIQUÉE POUR ESTIMER UN ÉTAT DE STRESS D'UNE PERSONNE**

(57) Procédé de détermination d'une fonction d'appartenance, la fonction d'appartenance permettant d'estimer un niveau de stress d'un utilisateur à partir d'une caractéristique physiologique mesurée sur l'utilisateur, la fonction d'appartenance variant, en fonction de la caractéristique physiologique, entre :
• une première valeur, représentative d'un état de repos ;
• et une deuxième valeur, représentative d'un état de stress ;
la fonction d'appartenance prenant en compte une fonction de répartition, préalablement définie, la fonction de répartition étant une fonction continue et monotone, la fonction de répartition étant appliquée à une caractéristique normalisée, établie à partir d'une caractéristique physiologique mesurée, le procédé comportant une détermination d'une fonction de normalisation, et éventuellement d'une fonction de standardisation, la fonction de normalisation et l'éventuelle fonction de standardisation étant déterminées à partir de caractéristiques physiologiques mesurées sur plusieurs individus de test, en différentes périodes de calibration. Figure d'abrégé : figure 3B.

Fig. 3A

Fig. 3B

EP 3 841 964 A1

**Description**

**DOMAINE TECHNIQUE**

Le domaine technique de l'invention est la détermination d'un niveau de stress d'un utilisateur à partir d'au moins une mesure d'une caractéristique physiologique effectuée sur l'utilisateur. La détermination du niveau de stress est établie en mettant en œuvre une fonction d'appartenance, découlant de la logique floue.

**DOMAINE TECHNIQUE**

Il est possible de déterminer un niveau de stress d'un utilisateur à partir de mesures d'un ou de plusieurs caractéristiques physiologiques de ce dernier. La caractéristique physiologique peut être une activité cardiaque, mesurée par exemple par un électrocardiogramme (ECG) ou une simple détermination d'une fréquence cardiaque, ou une activité musculaire, mesurée par un électromyogramme (EMG), ou encore une mesure de la conductance électrique de la peau. La publication Ollander "A comparison of wearable and stationary sensors for stress detection", 2016 IEEE international conférence on Systems, Man and Cybernetics (SMC), Oct 2016, décrit comment certains caractéristiques physiologiques peuvent être utilisés pour établir un indicateur de stress d'un utilisateur.

L'émergence de capteurs nomades et connectés, destinés à être portés par un utilisateur, permet d'accéder à des mesures de caractéristiques physiologiques de façon simple et peu couteuse. Il s'agit par exemple de capteurs spécifiques pouvant être fixés à un bracelet ou intégrés dans des montres ou reliés à des smartphones. Par exemple, le dispositif Empatica E4 comporte différents capteurs permettant d'accéder aisément à des caractéristiques physiologiques de type activité électrodermale, activité cardiaque ou température.

A partir des paramètres mesurés, des algorithmes de classification peuvent être mis en œuvre, de façon à déterminer si l'utilisateur se trouve dans un état de stress ou dans un état de repos. Certains algorithmes de classification sont basés sur la logique floue. Ce type d'algorithme est par exemple décrit dans la publication Kumar M "Fuzzy techniques for subjective workload-score modeling under uncertainties", IEEE transactions on systems, man and cybernetics- part B, Vol. 38, No. 6, December 2008. Ce type d'algorithme suppose l'établissement d'une phase d'apprentissage, au cours de laquelle un utilisateur est placé dans une situation de stress, ou dans diverses situations de stress. La publication De Santos Sierra A. et Al "Real-time stress détection by means of physiological signais", Recent Application in Biometrics (2011-07-27) décrit une approche analogue, selon laquelle au cours de l'apprentissage, l'utilisateur doit être successivement dans un état de stress ainsi que dans un état de repos. Le fait d'imposer un apprentissage, durant lequel l'utilisateur est placé dans une situation de stress, est contraignant. De plus, la fiabilité de telles méthodes peut être compromise par les variabilités physiologiques d'un utilisateur à un autre.

La demande de brevet FR1856504, déposée le 13/07/2018 décrit un procédé permettant de déterminer un niveau de stress d'un utilisateur à partir de caractéristiques physiologiques mesurés sur ce dernier. Un aspect intéressant de ce procédé est qu'il ne nécessite pas de placer l'utilisateur dans un état de stress lors de la calibration. Autrement dit, la calibration n'est effectuée que lorsque l'utilisateur est au repos. Cela permet une mise en œuvre particulièrement simple du procédé, à partir d'équipements de mesure portés par l'utilisateur. De plus, la calibration peut être renouvelée de façon périodique. Dans cette demande de brevet, le niveau de stress de l'utilisateur est déterminé selon les principes de la logique floue : une fonction d'appartenance est préalablement établie. Le niveau de stress est déterminé en fonction de l'image, par la fonction d'appartenance, de la valeur de la caractéristique physiologique mesurée. Ce procédé est également décrit dans la publication Charbonnier, Sylvie et al "A Multi-feature Fuzzy Index to Assess Stress Level from Bio-signals. Conférence proceedings : Annual International Conférence of the IEEE Engineering in Medicine and Biology Society". IEEE Engineering in Medicine and Biology Society. Conference. 2018. 1086-1089.

Les inventeurs ont estimé que le procédé décrit dans la demande FR1856504, ainsi que dans la publication précitée, pouvait être amélioré, de façon à augmenter la sensibilité ainsi que la spécificité de la détection d'un niveau de stress. C'est l'objet du procédé décrit par la suite.

**EXPOSE DE L'INVENTION**

Un premier objet de l'invention est un procédé de détermination d'une fonction d'appartenance, la fonction d'appartenance permettant d'estimer un niveau de stress d'un utilisateur à partir d'une caractéristique physiologique mesurée sur l'utilisateur, durant une période de mesure, la fonction d'appartenance variant, en fonction de la caractéristique physiologique, entre :

- une première valeur, représentative d'un état de repos ;
- et une deuxième valeur, représentative d'un état de stress ;

la fonction d'appartenance prenant en compte une fonction de répartition, préalablement définie, la fonction de répartition étant une fonction continue et monotone, la fonction de répartition étant appliquée à une caractéristique normalisée, établie à partir d'une caractéristique physiologique mesurée, le procédé comportant les étapes suivantes :

a) mesures de valeurs d'une caractéristique physiologique, en différentes périodes de calibration, sur au moins un individu de test, et de préférence plusieurs individus de test, chaque individu de test étant placé, en chaque période de calibration, soit dans un état de repos, soit dans un état de stress, soit dans un état intermédiaire, l'état intermédiaire étant un état dans lequel l'individu de test est considéré comme n'étant ni dans un état de stress, ni dans un état de repos;

b) éventuellement, application d'une fonction de standardisation aux valeurs mesurées au repos, lors de l'étape a), pour obtenir des paramètres standardisés, la fonction de standardisation étant concave;

c) à partir :

- des valeurs mesurées lors de l'étape a) et/ou éventuellement standardisées lors de l'étape b) ;
- de la fonction de répartition ;

détermination d'une fonction de normalisation, la fonction de normalisation étant destinée à être appliquée aux valeurs mesurées de la caractéristique physiologique, éventuellement standardisées, afin d'établir des valeurs normalisées, la détermination de la fonction de normalisation comportant :

- une optimisation d'un intervalle de repos, représentatif des valeurs de la caractéristiques mesurées lorsque chaque individu de test est au repos ;
- une optimisation d'une distance seuil, entre l'intervalle de repos, et un intervalle de stress, représentatif des valeurs de la caractéristique mesurées lorsque chaque individu de test est dans un état de stress;

d) détermination de la fonction d'appartenance associée à la caractéristique physiologique, la fonction d'appartenance correspondant à une composition de la fonction de répartition, de la fonction de normalisation et de l'éventuelle fonction de standardisation.

Le rôle de la fonction de normalisation est de distribuer les valeurs mesurées de la caractéristique physiologique, éventuellement standardisées, de façon optimale de façon à optimiser, après application de la fonction de répartition, l'estimation du niveau de stress de l'individu.

Selon un mode de réalisation, lors de l'étape a), les valeurs de la caractéristique physiologique mesurées alors que chaque individu de test est dans un état de stress, de repos ou intermédiaire sont regroupées respectivement une classe de stress, une classe de repos et une classe intermédiaire. Lors de l'étape c),

- l'intervalle de repos est optimisé de façon à optimiser une classification, par la fonction de répartition, de valeurs normalisées, correspondant respectivement à la classe de repos et à une union de la classe intermédiaire et de la classe de stress;
- la distance seuil est optimisée de façon à optimiser une classification, par la fonction de répartition, de valeurs normalisées correspondant respectivement à la classe de stress et une union de la classe intermédiaire et de la classe de repos.

L'étape c) peut comporter une détermination :

- d'un indicateur de classification des valeurs normalisées entre la classe de repos et une union de la classe intermédiaire et de la classe de stress, l'indicateur de classification étant basé sur une proportion de faux positifs et de faux négatifs ;
- d'un indicateur de classification des valeurs normalisées entre la classe de stress et une union de la classe intermédiaire et de la classe de repos, l'indicateur de classification étant basé sur une proportion de faux positifs et de faux négatifs.

L'étape c) peut comporter :

- une détermination d'un intervalle de repos optimal, en fixant la distance seuil;
- puis une détermination de la distance seuil optimale, en fonction de l'intervalle de repos optimal.

L'étape c) peut comporter une optimisation d'une étendue de l'intervalle de repos.

L'étape c) peut comporter une optimisation d'un facteur d'échelle, la distance seuil étant déterminée en multipliant le facteur d'échelle par l'étendue de l'intervalle de repos.

Selon un mode de réalisation, la fonction de normalisation est une fonction affine, l'étape c) comportant une détermination des paramètres de la fonction affine à partir de l'intervalle de repos et de la distance seuil optimisés.

La fonction de standardisation peut être de type logarithmique ou racine carrée.

Selon un mode de réalisation, le procédé comporte :

- suite à l'étape a), calcul d'un coefficient d'asymétrie d'une distribution des valeurs de la caractéristique physiologique mesurées lorsque chaque individu de test est au repos, le coefficient d'asymétrie quantifiant l'asymétrie de la distribution ;
- comparaison du coefficient d'asymétrie par rapport à un seuil prédéterminé;
- en fonction de la comparaison, mise en œuvre de l'étape b).

Selon un mode de réalisation :

- les étapes a) à d) forment un enchainement d'étapes associé à la caractéristique physiologique mesurée lors de l'étape a), et conduisant à la détermination d'une fonction d'appartenance associée à la caractéristique physiologique ;
- le procédé comporte différents enchainements d'étapes a) à d), respectivement associés à différentes caractéristiques physiologiques, de façon à déterminer différentes fonctions d'appartenance, chaque fonction d'appartenance étant respectivement associée à une caractéristique physiologique.

Un deuxième objet de l'invention est un procédé de détermination d'un niveau de stress d'un utilisateur, en fonction d'une caractéristique physiologique mesurée sur l'utilisateur, durant une période de mesure, dont la valeur est susceptible de varier en fonction du niveau de stress de l'utilisateur, le procédé comportant les étapes suivantes :

i) mesure d'une valeur de la caractéristique physiologique durant une période de mesure;
ii) application d'une fonction d'appartenance à la valeur de la caractéristique physiologique mesurée lors de l'étape i) ;
iii) estimation du niveau de stress de l'utilisateur en fonction de la valeur de la fonction d'appartenance calculée lors de l'étape ii) ;

le procédé étant caractérisé en ce que la fonction d'appartenance est une fonction déterminée selon un procédé selon le premier objet de l'invention.

Un troisième objet de l'invention est un procédé de détermination d'un niveau de stress d'un utilisateur, en fonction d'une pluralité de caractéristiques physiologiques de l'utilisateur, la valeur de chaque caractéristique physiologique étant susceptible de varier en fonction du niveau de stress de l'utilisateur, le procédé comportant les étapes suivantes :

i) mesure de valeurs de différentes caractéristiques physiologiques durant une période de mesure ;
ii) pour chaque valeur de caractéristique physiologique mesurée lors de l'étape i), application d'une fonction d'appartenance associée à la caractéristique physiologique
iii) estimation du niveau de stress en combinant les valeurs des fonctions d'appartenance calculées lors de l'étape i) ;

le procédé étant caractérisé en ce qu'au moins une fonction d'appartenance, associée à un caractéristique physiologique, est une fonction déterminée selon un procédé selon le premier objet de l'invention.

Le procédé selon le deuxième ou le troisième objet de l'invention peut comporter comportant une phase d'initialisation, regroupant différentes périodes d'initialisation, au cours desquelles l'utilisateur est considéré comme étant dans un état de repos, la phase d'initialisation comportant :

- une mesure, en chaque période d'initialisation, d'une caractéristique physiologique;
- une détermination d'un indicateur de dispersion des valeurs mesurées à chaque période d'initialisation ;

le procédé comportant, préalablement aux étapes i) à iii), un ajustement de la fonction d'appartenance, établie pour la caractéristique physiologique, en prenant en compte l'indicateur de dispersion.

L'invention sera mieux comprise à la lecture de l'exposé des exemples de réalisation présentés, dans la suite de la description, en lien avec les figures listées ci-dessous.

## FIGURES

La figure 1 représente un dispositif permettant une mise en œuvre de l'invention.

La figure 2 illustre les principales étapes d'un mode de réalisation de l'invention.

La figure 3A schématise les principales étapes d'une phase de calibration, permettant de déterminer la fonction d'appartenance.

Les figures 3B et 3C illustrent le cas où la variation de la caractéristique par rapport au niveau de stress est respectivement positive ou négative.

La figure 3D illustre une hiérarchie entre des paramètres définissant une fonction de normalisation.

La figure 4A montre une distribution, considérée comme symétrique, de valeurs d'une caractéristique physiologique mesurée au cours d'une phase de calibration, alors que chaque individu de test est au repos.

La figure 4B montre une distribution, considérée comme non symétrique, de valeurs d'une autre caractéristique physiologique mesurée au cours d'une phase de calibration, alors que chaque individu de test est au repos.

La figure 4C montre une distribution obtenue après une application d'une fonction de standardisation aux valeurs dont la distribution représentée sur la figure 4B. La comparaison entre les figures 4B et 4C illustre l'effet de la fonction de standardisation.

Les figures 5A et 5B montrent respectivement l'optimisation de l'étendue de l'intervalle de repos et l'optimisation d'un facteur d'échelle.

La figure 6A représente une application de la fonction d'appartenance en prenant en compte un facteur d'échelle nul.

La figure 6B représente une application de la fonction d'appartenance en prenant en compte un facteur d'échelle ayant fait l'objet d'une optimisation.

La figure 7 montre les étapes d'un procédé de détermination d'un niveau de stress d'un utilisateur, en utilisant au moins une fonction d'appartenance telle que déterminée selon les étapes schématisées sur la figure 3A.

## EXPOSE DE MODES DE REALISATION PARTICULIERS

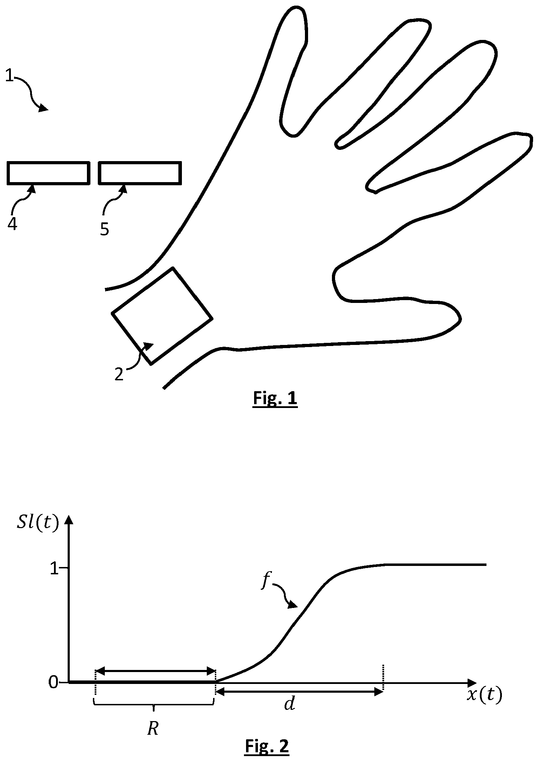

On a représenté, sur la figure 1, un dispositif 1 permettant la mise en œuvre de l'invention. Un capteur 2 est disposé contre le corps d'un utilisateur, par exemple au niveau du poignet. Le capteur 2 est configuré pour mesurer un signal physiologique, durant une période de mesure $t$, à partir duquel on établit une caractéristique physiologique $x(t)$ de l'utilisateur durant la période de mesure $t$. Le terme période désigne un intervalle temporel, par exemple quelques secondes ou quelques minutes. La valeur de la caractéristique physiologique considérée peut varier en fonction d'un niveau de stress de l'utilisateur. La caractéristique physiologique x peut être une caractéristique telle que décrite dans les publications citées dans l'art antérieur, en étant désigné par le terme anglais "feature". La caractéristique physiologique peut être par exemple :

- une caractéristique liée à une activité cardiaque de l'utilisateur, par exemple liée à la fréquence cardiaque. Une telle caractéristique peut être mesurée par des électrodes similaires à des électrodes d'un électrocardiogramme (ECG), ou par des moyens optiques, de type photoplethysmographie (PPG).
La caractéristique physiologique peut être une moyenne de la fréquence cardiaque (HR) ou un paramètre de dispersion de la fréquence cardiaque tel que l'écart-type, calculés sur une période temporelle prédéterminée. La caractéristique physiologique peut résulter d'une analyse fréquentielle de l'activité cardiaque dont celle de l'intervalle inter-battements, connu de l'homme du métier sous la désignation IBI (Inter Beat Interval). Sur un ECG, cet intervalle peut s'étendre entre deux pics R de deux impulsions cardiaques consécutives. L'analyse fréquentielle peut être effectuée dans des plages de fréquences comprises entre 0 Hz et 0.5 Hz, et plus spécifiquement, par exemple entre 0 et 0.003 Hz (ultra basses fréquences), ou entre 0.003 et 0.04 Hz (très basses fréquences), entre 0.04 Hz et 0.15 Hz (basses fréquences), ou entre 0.15 Hz et 0.5 Hz (hautes fréquences).

- Une caractéristique liée à une activité musculaire de l'utilisateur, ce type de caractéristique pouvant être déterminée à partir de signaux mesurés par électromyographie (EMG).

- Une caractéristique liée à l'activité électrodermale de l'utilisateur, représentative de propriétés de conduction électrique ou d'impédance électrique de la peau (par exemple la conductance ou l'impédance électrodermale) et/ou de propriétés capacitives de la peau. Il peut s'agir d'une valeur moyenne de la conductance de la peau ou de la moyenne de valeur absolue d'une dérivée de la conductance de la peau. Il peut également s'agir d'une fréquence, ou d'une durée moyenne ou d'une amplitude moyenne des réponses électrodermales calculées durant une période temporelle prédéterminée. Plus généralement, la caractéristique considérée est une caractéristique d'une réponse électrodermale.

- Une température de la peau de l'utilisateur.

- Une mesure d'un mouvement, par exemple d'une accélération selon au moins un axe, une telle mesure permettant de caractériser un tremblement ou une activité physique susceptible d'impacter la physiologie de l'utilisateur. La caractéristique considérée peut être une norme de l'accélération.

- Une mesure d'une activité corticale, par exemple à partir d'électrodes de type électroencéphalographie (EEG). Il peut par exemple s'agir d'une puissance spectrale dans une bande de fréquence prédéterminée calculée sur une période temporelle prédéterminée.

La caractéristique physiologique mesurée peut être représentative de l'activité cardiaque. Si $HR_j$ correspond à la fréquence cardiaque mesurée en un instant $j$, la caractéristique physiologique $x(t)$ à l'instant $t$ peut être telle que :

$$x(t) = \sqrt{\frac{1}{N_j}\sum_j \left(HR_j - HR_{j-1}\right)^2} \quad (1) \ ,$$

où $N_j$ correspond aux nombres de mesures de fréquence cardiaque prises en compte, avec : $t - N_j \leq j \leq t$. $N_j$ est dimensionné pour prendre en compte les mesures de fréquence cardiaque durant une durée glissante de quelques secondes ou quelques dizaines de secondes, voire quelques minutes, par exemple 60s.

Si $IBI_j$ correspond à l'intervalle inter-battements mesuré en un instant $j$, la caractéristique physiologique x(t) à l'instant $t$ peut être telle que :

$$x(t) = \sqrt{\frac{1}{N_j}\sum_j \left(IBI_j - IBI_{j-1}\right)^2} \quad (2)$$

où $N_j$ correspond aux nombres de mesures de fréquence cardiaque prises en compte,
avec : $t - N_j \leq j \leq t$. $N_j$ est dimensionné pour prendre en compte les mesures de fréquence cardiaque durant une durée glissante de quelques secondes, ou quelques dizaines de secondes, voire quelques minutes, par exemple 60s.

Les caractéristiques physiologiques décrites dans les deux paragraphes qui précèdent conviennent particulièrement bien à une mise en œuvre de l'invention. Elles peuvent être utilisées seules ou être combinées, comme décrit par la suite. Une caractéristique physiologique est déterminée, pour une période de mesure, à partir de signaux mesurés durant la période de mesure. Elle peut résulter d'une analyse statistique ou fréquentielle de ces signaux.

L'objectif de l'invention est de déterminer un niveau de stress $SI(t)$ d'un utilisateur à différentes périodes de mesure $t$. L'utilisateur est un individu vivant, par exemple un être humain ou un animal.

Le capteur 2 est relié à un microprocesseur 4, ce dernier étant relié à une mémoire 5 dans laquelle sont stockées des instructions pour mettre en œuvre le procédé décrit ci-après. Le microprocesseur 4 reçoit les mesures du capteur 2, par une liaison filaire ou une liaison sans fil. Le microprocesseur 4 peut être porté par l'utilisateur, en étant disposé au niveau du capteur ou en étant implanté dans un dispositif annexe porté par l'utilisateur, par exemple un objet nomade tel un smartphone. Le microprocesseur 4 peut également être distant de l'utilisateur.

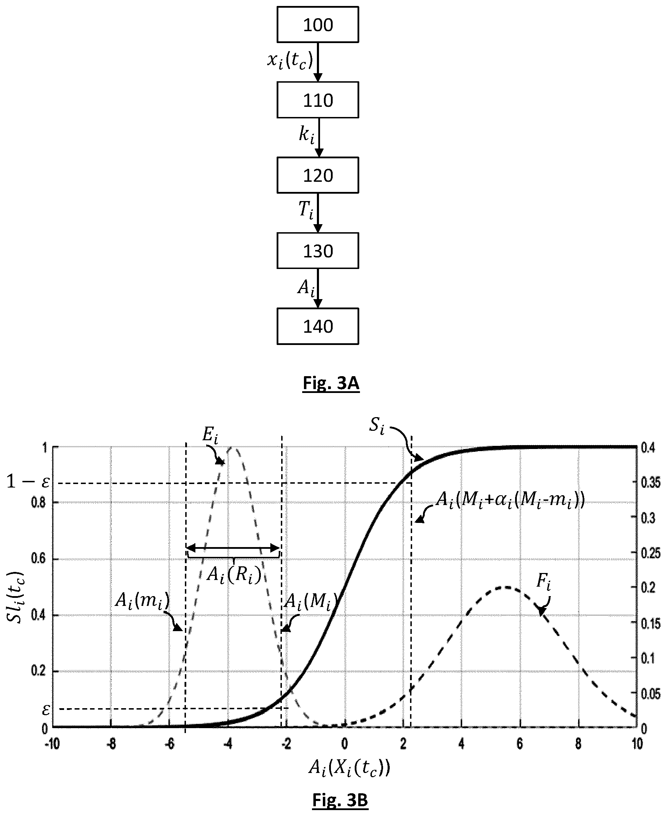

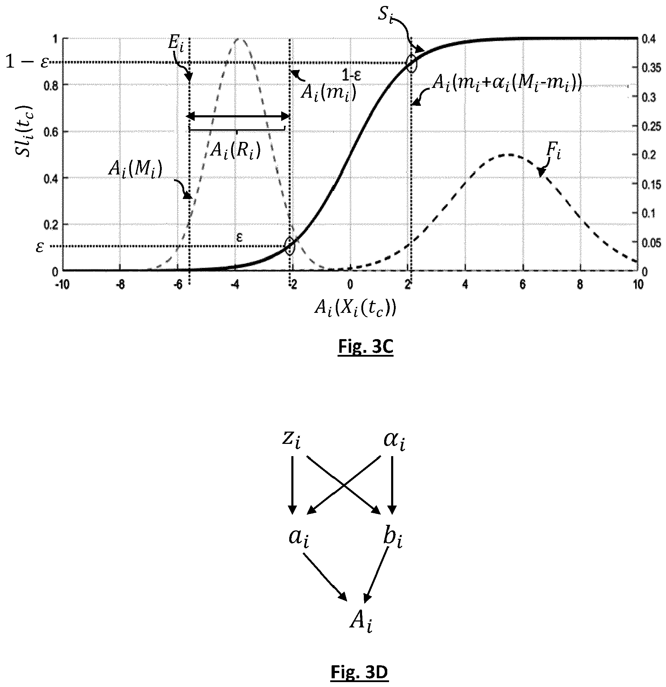

Le fonctionnement du dispositif 1 est décrit dans la demande de brevet FR1856504, et plus particulièrement dans les étapes 130 et 140 et la figure 3 de cette dernière. D'une façon générale, le dispositif met en œuvre un procédé répété en différentes périodes de mesure $t$. L'objectif est d'estimer un niveau de stress $SI(t)$ correspondant à chaque période de mesure. Au cours de chaque période, une caractéristique physiologique $x(t)$, telle que précédemment définie, est déterminée. Le procédé comporte une application d'une fonction d'appartenance $f$, de telle sorte que le niveau de stress $SI(t)$, durant la période de mesure, correspond à l'image, par la fonction d'appartenance, de la caractéristique physiologique. Autrement dit, $SI(t) = f(x(t))$

La fonction d'appartenance $f$ est destinée à estimer un niveau de stress $SI$ à partir de la valeur d'une caractéristique physiologique $x(t)$ mesurée à une période de mesure $t$. Le niveau de stress $SI$ peut par exemple varier entre 0 et 1, 0 correspondant à un état de repos et 1 correspondant à un état de stress de l'utilisateur. Selon les principes de la logique floue, la fonction d'appartenance $f$ peut définir des niveaux intermédiaires, compris entre 0 et 1, et correspondant à un état dans lequel l'utilisateur n'est ni en état de stress, ni au repos. La fonction d'appartenance $f$ est de préférence continue dans un espace de départ $\mathbb{E}$ défini par les valeurs susceptibles d'être prises par la caractéristique physiologique mesurée. L'espace de départ $\mathbb{E}$ peut par exemple être l'ensemble des réels positifs. Ainsi, $f$: $\mathbb{E} = \mathbb{R}^+ \to [0,1]$ et $f(x(t)) = Sl\ (t)$. La fonction d'appartenance $f$ peut par exemple être linéaire par morceau, ou prendre une forme tangente hyperbolique ou sigmoide.

La figure 2 représente un exemple selon lequel la fonction d'appartenance $f$ est de type sigmoïde. Une fonction sigmoïde est de type :

$$f(x) = \frac{1}{1 + e^x} \quad (3)$$

Lorsque les valeurs de la caractéristique $x(t)$ sont comprises dans un intervalle de repos $X_r$, le niveau de stress $Sl\ (t)$ est proche de 0 ou égal à 0. L'utilisateur est considéré comme étant au repos. Lorsque les valeurs de la caractéristique $x(t)$ sont distantes, d'une distance inférieure à une distance seuil $d$, de l'intervalle de repos $R$, l'utilisateur est considéré comme étant dans un état intermédiaire, entre l'état de repos et un état de stress : $0 < Sl\ (t) < 1$. Lorsque les valeurs de la caractéristique $x(t)$ sont distantes, d'une distance supérieure à une distance seuil $d$, de l'intervalle de repos, l'utilisateur est considéré comme étant dans un état de stress. $Sl\ (t)$ est alors égal à 1 ou proche de 1.

L'exemple donné en lien avec la figure 2 est valable lorsque la fonction d'appartenance $f$ est croissante, c'est-à-dire que le niveau de stress est d'autant plus élevé que la valeur mesurée de la caractéristique physiologique l'est également. Dans certains cas de figure, par exemple lorsque la caractéristique considérée est la résistance de la peau, la fonction d'appartenance $f$ est décroissante : le niveau de stress est d'autant plus élevé que la valeur de la caractéristique mesurée est faible

Selon une possibilité, la méthode décrite ci-dessus peut être appliquée en mesurant, à chaque période de mesure $t$, différentes caractéristiques physiologiques $x_i$, l'indice $i$ identifiant la caractéristique considérée, avec $1 \le i \le I$, $I$ désignant le nombre de caractéristiques physiologiques considérées. Une fonction d'appartenance $f_i$ est alors définie relativement à chaque caractéristique physiologique $x_i$. Les fonctions d'appartenance $f_i, f_{i+1}$ respectivement associées à de deux caractéristiques différentes $x_i, x_{i+1}$, peuvent être établies indépendamment l'une de l'autre. Il est cependant préférable que pour chaque fonction d'appartenance, l'état de repos et l'état de stress correspondent respectivement aux mêmes niveaux, par exemple 0 pour l'état de repos et 1 pour l'état de stress. On aboutit ainsi à une définition de $I$ fonctions d'appartenance $f_1 ... f_I$, respectivement associées aux $I$ caractéristiques physiologiques mesurées $x_1 ... x_I$.

Après que chaque fonction d'appartenance $f_i$ a été définie, le procédé comporte, à chaque période de mesure $t$, son application pour chaque caractéristique $x_i(t)$ mesurée, de façon à déterminer un niveau de stress $Sl_i(t)$ associé à chaque caractéristique $x_i(t)$, selon l'expression $Sl_i(t) = f_i(x_i(t))$. On aboutit ainsi à une définition de $I$ niveaux de stress $Sl_1(t) ... Sl_I(t)$, respectivement associées aux $I$ caractéristiques physiologiques considérées $x_1 ... x_I$.

Les différents niveaux de stress $Sl_1(t) ... Sl_I(t)$, respectivement associés à chaque caractéristique $x_i(t)$, sont combinés, de façon à déterminer un niveau de stress global, ou multiparamétrique, $Sl(t)$, selon les principes de la logique floue. La combinaison peut être un calcul d'une valeur moyenne ou d'une valeur médiane. Il peut également s'agir d'une moyenne pondérée, dans laquelle chaque niveau de stress $Sl_i(t)$ est affecté d'un facteur de pondération $\lambda_i$ dépendant de l'importance que l'on souhaite attribuer à la caractéristique physiologique $x_i$ relativement aux autres caractéristiques considérées. La combinaison des différents niveaux de stress $Sl_1(t) ... Sl_I(t)$ peut être effectuée en appliquant des règles d'inférence prédéterminées. A partir du niveau de stress multiparamétrique $Sl(t)$, on peut déterminer si l'utilisateur se trouve dans un état de stress.

Les inventeurs ont considéré que la fonction d'appartenance devait être raffinée, de façon à prendre en compte un traitement des valeurs mesurées de chaque caractéristique physiologique. Cela est particulièrement pertinent lorsque différentes caractéristiques physiologiques $x_i$ sont adressées lors de chaque période de mesure $t$, de façon à obtenir un niveau de stress multiparamétrique. L'objectif du traitement est d'améliorer les performances de la détermination du niveau de stress, et notamment la sensibilité (c'est-à-dire une proportion minimale de faux négatifs), ainsi que la spécificité (c'est-à-dire une proportion minimale de faux positifs).

Le traitement des valeurs mesurées consiste à appliquer une fonction d'appartenance à la valeur d'une caractéristique physiologique mesurée au cours de chaque période de mesure. L'invention se distingue de l'art antérieur en ce que la fonction d'appartenance est une composition de différentes fonctions, définies au cours d'une phase de calibration, décrite ci-après. Plus précisément, pour chaque caractéristique physiologique considérée $x_i$, une fonction d'appartenance $f_i$ est définie au cours de la phase de calibration. Chaque fonction d'appartenance $f_i$ associée à une caractéristique physiologique $x_i$, correspond à une composition :

- éventuellement d'une fonction de standardisation $T_i$. La fonction de standardisation est généralement déterminée au cours de la phase de calibration. La fonction de standardisation est déterminée de telle sorte que lorsqu'un utilisateur est au repos, les valeurs $x_i(t)$ de la caractéristique physiologique $x_i$ sont distribuées par une distribution se rapprochant d'une distribution symétrique, par exemple gaussienne. L'application de la fonction de standardisation à une valeur mesurée $x_i(t)$ de la caractéristique physiologique $x_i$ conduit à l'obtention d'une valeur standardisée $T_i(x_i(t))$. Lorsque le procédé se base sur une mesure de différentes caractéristiques physiologiques $x_1$, $x_2$ durant une période de mesure $t$, les fonctions de standardisation $T_1$, $T_2$ respectivement associées aux caractéristiques $x_1$, $x_2$ sont généralement différentes.

- d'une fonction de normalisation $A_i$, établie durant la phase de calibration. La fonction de normalisation $A_i$ permet de distribuer les valeurs mesurées $x_i(t)$ de la caractéristique physiologique, ou des valeurs standardisées $T_i(x_i(t))$, de façon optimale relativement à la spécificité et à la sensibilité. L'application de la fonction de normalisation $A_i$ à une valeur mesurée $x_i(t)$ de la caractéristique physiologique $x_i$, ou à une valeur standardisée $T_i(x_i(t))$, permet d'obtenir une valeur normalisée $A_i(x_i(t))$ ou $A_i(T_i(x_i(t)))$. Lorsque le procédé se base sur une mesure de différentes caractéristiques physiologiques $x_1$, $x_2$ durant une période de mesure $t$, les fonctions de normalisation $A_1$, $A_2$ respectivement associées aux caractéristiques $x_1$, $x_2$ sont généralement différentes.

- d'une fonction de répartition $S_i$, prédéfinie. La fonction de répartition $S_i$, appliquée à une valeur normalisée $A_i(x_i(t))$ ou $A_i(T_i(x_i(t)))$, permet d'estimer un niveau de stress $SI_i(t)$ associé à la caractéristique physiologique $x_i$. La fonction de répartition est de préférence monotone, et de préférence continue. La fonction de répartition $S_i$ peut par exemple être linéaire par morceau, ou prendre une forme tangente hyperbolique ou sigmoide. Lorsque le procédé se base sur une mesure de différentes caractéristiques physiologiques $x_1$, $x_2$ durant une période de mesure $t$, les fonctions de répartition $S_1$, $S_2$ respectivement associées aux caractéristiques $x_1$, $x_2$ peuvent être différentes ou identiques.

Ainsi, le procédé met en œuvre, pour chaque caractéristique physiologique $x_i$ considérée, une fonction d'appartenance $f_i$ formée par une composition de l'éventuelle fonction de standardisation $T_i$, avec la fonction de normalisation $A_i$ et la fonction de répartition $S_i$. Ainsi,

$$f_i = S_i \circ A_i \circ T_i \quad (4)$$

ou

$$f_i = S_i \circ A_i \quad (5)$$

$\circ$ désigne l'opérateur de composition de fonctions.

La fonction de normalisation $A_i$ permet une projection des caractéristiques physiologiques mesurées, pour un utilisateur donné, dans un espace comportant les antécédents de la fonction de répartition. La fonction de normalisation $A_i$ est optimisée de façon à optimiser l'estimation du niveau de stress, après application de la fonction de répartition $S_i$, en considérant en particulier la spécificité et la sensibilité.

On va à présent décrire, en lien avec la figure 3A, les principales étapes de la phase de calibration, permettant de définir les fonctions de standardisation et de normalisation en prenant en compte une fonction de répartition prédéterminée. La phase de calibration est effectuée sur une population test, formée d'au moins un individu de test, et de préférence de plusieurs individus de test. Au cours de cette phase, chaque individu de test de la population de test suit un protocole, de façon à être considéré soit dans un état de repos, soit dans un état de stress, soit dans un état intermédiaire, c'est-à-dire ni en repos, ni en stress. L'état intermédiaire peut par exemple correspondre à un état dans lequel l'individu de test est actif, sans être stressé : il effectue par exemple une activité physique, et/ou intellectuelle, sans que cette activité soit considérée comme stressante. Chaque individu de test est considéré comme représentatif de l'utilisateur destiné à utiliser le dispositif.

Des protocoles connus de l'homme du métier permettent de considérer qu'un individu est dans un état de stress. Il s'agit par exemple d'un protocole usuellement désigné par l'acronyme "MST" (Mental Stress Test), au cours duquel un individu doit compter, à rebours, en partant d'un nombre prédéterminé, le comptage étant effectué par incréments. L'individu est filmé, et l'essai est réalisé en présence de deux observateurs. Par exemple, l'individu doit compter à rebours à partir de 1022 par incréments de 13. Il peut également s'agir d'un protocole usuellement désigné par l'acronyme "TSST" (Trier Social Stress Test), au cours duquel l'individu est filmé durant une simulation d'entretien de recrutement devant deux observateurs. Au cours de chaque protocole, une ou plusieurs caractéristiques physiologiques sont mesurés, au cours de différentes périodes. Durant la phase de calibration, les périodes $t_c$ au cours desquelles des caractéristiques physiologiques sont mesurées sont appelées périodes de calibration.

Les étapes 100 à 140 décrites par la suite sont relatives à une caractéristique physiologique $x_i$. Lorsque le procédé met en œuvre différentes caractéristiques physiologiques, les étapes 100 à 140 sont effectuées en parallèle, pour chaque caractéristique physiologique adressée.

<u>Etape 100</u> : mesure de valeurs $x_i(t_c)$ de la caractéristique physiologique $x_i$ durant plusieurs périodes de calibration $t_c$, et cela pour un ou plusieurs individus de test. Les mesures sont effectuées alors que chaque individu de test est dans un état considéré comme connu. La population et le nombre de mesures sont dimensionnés de façon à obtenir un échantillonnage statistiquement représentatif de chaque caractéristique, et dans chaque état (état de stress, état de repos, état intermédiaire). Typiquement, on dispose d'au moins 30 valeurs mesurées pour chaque caractéristique et dans chaque état.

Etape 110 : Etude de la symétrie des valeurs mesurées lors de l'étape 100, alors que chaque individu de test est au repos.

Pour la caractéristique physiologique $x_i$, on détermine une distribution $D_i$ des valeurs $x_i(t_c)$ mesurées alors que chaque individu de test est au repos. Un coefficient de symétrie $k_i$ est calculé pour la caractéristique physiologique. Le coefficient de symétrie $k_i$ quantifie la symétrie de la distribution $D_i$. Par exemple, le coefficient de symétrie $k_i$ est tel que :

$$k_i = \frac{E[x_i(t_c) - \mu_i]^3}{\sigma_i^{\,3}} \quad (6)$$

où $\mu_i$ et $\sigma_i$ sont respectivement la moyenne et l'écart type de la distribution $D_i$. $E$ est l'opérateur Espérance. Lorsque $k_i$ est positif, la distribution $D_i$ est centrée sur les valeurs les plus basses de la caractéristique $x_i$. Lorsque $k_i$ est négatif, la distribution $D_i$ est centrée sur les valeurs les plus hautes de la caractéristique physiologique $x_i$. Plus la valeur absolue de $k_i$ est faible, plus la distribution $D_i$ est considérée comme symétrique.

Suite à l'étape 110, on dispose d'un coefficient de symétrie $k_i$ correspondant à la distribution $D_i$ des valeurs $x_i(t_c)$ de la caractéristique physiologique $x_i$ mesurées au cours de la phase de calibration, lorsque chaque individu test est au repos.

Etape 120 : Détermination d'une fonction de standardisation

Cette étape est facultative. Elle est de préférence mise en œuvre lorsque suite à l'étape 110, la valeur absolue $|k_i|$ du coefficient de symétrie $k_i$ d'une distribution $D_i$ dépasse un certain seuil $k_{th}$ : la distribution $D_i$ est alors considérée comme non symétrique. Une fonction de standardisation $T_i$ est appliquée à chaque valeur $x_i(t_c)$, de telle sorte que la distribution $D'_i$ des données $T_i(x_i(t_c))$, obtenues suite à l'application de la fonction de standardisation, présente un coefficient de symétrie $k'_i$ dont la valeur absolue est inférieure à la valeur absolue de $k_i$ : $|k'_i| < |k_i|$. De préférence, $|k'_i| < k_{th}$, de telle sorte que la distribution $D'_i$ est considérée comme symétrique.

La fonction de standardisation $T_i$ est de préférence une fonction concave. Il peut par exemple s'agir d'une fonction logarithmique, par exemple logarithmique en base 10, ou une fonction racine carrée. Quelle que soit le type de fonction de standardisation considéré (logarithmique, racine carrée ou autre), la fonction de standardisation est paramétrée pour minimiser la valeur absolue du coefficient de symétrie de la distribution $D'_i$. Ainsi,

$$T_i = \underset{T_i}{\mathrm{argmin}}(|k'_i|) \quad (7)$$

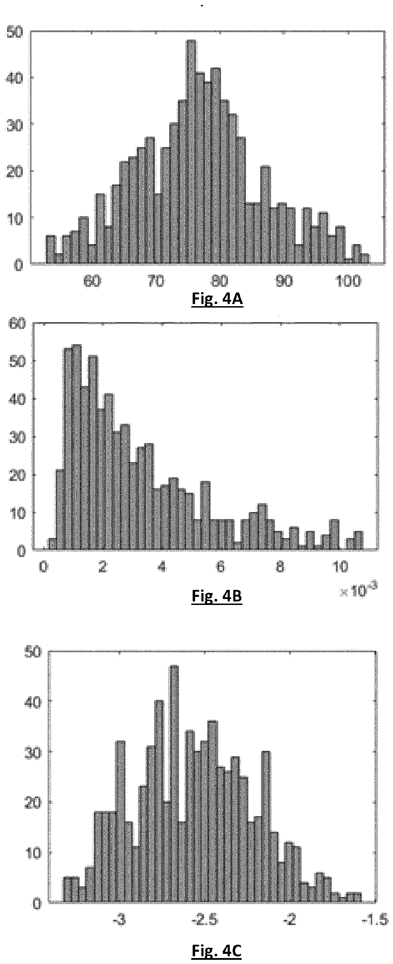

La figure 4A montre une distribution statistique obtenue expérimentalement, en considérant une valeur moyenne du rythme cardiaque de 20 individus de test en état de repos. Le coefficient de symétrie d'une telle distribution est proche de 0.

La figure 4B montre une distribution statistique obtenue expérimentalement, en considérant une puissance spectrale des intervalles inter-battements de 20 individus de test en état de repos. Le coefficient de symétrie d'une telle distribution est proche de 2. La distribution est fortement asymétrique. Une fonction logarithmique en base 10 a été appliquée aux valeurs formant la distribution statistique représentée sur la figure 4B. La figure 4C montre la distribution des valeurs ainsi standardisées. La fonction de standardisation, en l'occurrence la fonction logarithmique en base 10, permet d'obtenir une distribution symétrique des données standardisées.

Suite à l'étape 120, on dispose de valeurs mesurées, ou de valeurs mesurées puis standardisées, dont la distribution est symétrique. Cela facilite la mise en œuvre de l'étape 130. En effet, suite à l'application de la fonction de standardisation, les données correspondant à l'état de repos sont regroupées, de façon symétrique, autour d'une valeur centrale, dont l'occurrence est maximale, comme représenté sur les figures 3B et 3C.

Etape 130 : Détermination d'une fonction de normalisation.

L'objectif de la fonction de normalisation est de traiter des valeurs de telle sorte que l'image, par la fonction de répartition $S_i$, des valeurs traitées par la fonction de normalisation $A_i$, conduise à une séparation optimale entre les différents états, et en particulier entre les états de stress et les états de non stress. Cette étape suppose que la fonction de répartition $S_i$ soit connue. Comme précédemment indiqué, la fonction de répartition peut être une fonction sigmoïde.

Selon cet exemple :

- lorsque, pour la caractéristique $x_i$ considérée, une fonction de standardisation $T_i$ a été déterminée au cours de l'étape 120, la fonction de normalisation $A_i$ s'applique aux valeurs standardisées, de telle sorte que :

$$S_i\left(A_i\left(T_i\left(x_i(t_c)\right)\right)\right) = \frac{1}{1 + e^{\left(A_i\left(T_i(x_i(t_c))\right)\right)}} \quad (10)$$

- lorsque, pour la caractéristique $x_i$ considérée, une fonction de standardisation n'a pas été déterminée au cours de l'étape 120, la fonction de normalisation $A_i$ s'applique aux valeurs mesurées, de telle sorte que :

$$S_i\left(A_i\left(x_i(t_c)\right)\right) = \frac{1}{1 + e^{A_i(x_i(t_c))}} \quad (11)$$

Dans la suite de l'exemple, on désigne par $X_i(t_c)$ la variable d'entrée traitée par la fonction de normalisation $A_i$, résultant de l'étape 120 :

- en présence d'une standardisation : $X_i(t_c) = T_i(x_i(t_c))$ ;
- sans standardisation : $X_i(t_c) = x_i(t_c)$.

Sur la figure 3B, on a représenté une fonction de répartition $S_i$, s'appliquant à des valeurs $A_i(X_i(t_c))$, représentées sur l'axe des abscisses. L'axe des ordonnées (à droite) correspond à $S_i(A_i(X_i(t_c)))$, ce qui, selon l'expression (4), correspond à l'indice de stress $SI_i(t_c)$ calculé. Lors de cette étape, les données traitées sont issues de la phase de calibration. Aussi, à chaque période de calibration $t_c$, l'indice de stress $SI_i(t_c)$ est connu. L'exemple de la figure 3B correspond à une variation positive de la caractéristique par rapport au niveau de stress : cela signifie qu'un état de stress correspond à une augmentation de la valeur de la caractéristique.

La forme de la fonction de normalisation $A_i$ est prédéterminée. Cette dernière est paramétrée par des paramètres $\theta_i$. L'étape 130 vise à déterminer les paramètres $\theta_i$ de la fonction de normalisation $A_i$. Dans cet exemple, la fonction de normalisation $A_i$ est une fonction affine de type $A_i(X_i) = a_iX_i + b_i$ (12'). Les paramètres de la fonction sont $\theta_i = (a_i, b_i)$.

Une première propriété de la fonction de normalisation $A_i$ est que l'image, par cette dernière, des données d'entrée $X_i(t_c)$, résultant de la phase de calibration et correspondant à un état de repos, et regroupées dans un intervalle de repos $R_i$, forment des antécédents d'un état de repos $SI_{i,rest}$, après l'application de la fonction de répartition $S_i$. Ainsi :

$$X_i(t_c) \in R_i \Leftrightarrow S_i\left(A_i\left(X_i(t_c)\right)\right) \cong SI_{i,rest} \quad (12)$$

Le terme $\cong$ signifie égal à un terme d'incertitude $\varepsilon$ près. Le terme d'incertitude $\varepsilon$ est préalablement défini.

Le terme $SI_{i,rest}$ correspond à la valeur de l'indicateur de stress correspondant à un état de repos. Dans l'exemple représenté sur la figure 3B, la fonction de répartition $A_i$ est telle que l'indicateur de stress prend une valeur minimale $SI_{i,rest}$ lorsque l'utilisateur est au repos, et une valeur maximale $SI_{i,stress}$ lorsque l'utilisateur est en état de stress. Dans l'exemple représenté sur la figure 3B, exemple, $SI_{i,rest} = 0$ et $SI_{i,stress} = 1$. Ainsi, l'expression (12) devient :

$$X_i(t_c) \in R_i \Leftrightarrow S_i\left(A_i\left(X_i(t_c)\right)\right) < \varepsilon \quad (13)$$

De façon alternative, la variation par rapport au stress est négative : cela signifie qu'un état de stress correspond à une diminution de la caractéristique mesurée. Une telle alternative est représentée sur la figure 3C.

Les données d'entrée $X_{i,rest}(t_c)$ correspondant à un état de repos, sont comprise entre une borne minimale $m_i$ et une borne maximale $M_i$, délimitant l'intervalle $R_i$. Lors de la normalisation, les valeurs normalisées $A_i(X_i(t_c))$ sont telles que:

$$A_i(m_i) \leq A_i\left(X_{i,rest}(t_c)\right) \leq A_i(M_i) \quad (13')$$

Du fait que la distribution des données d'entrée $X_i(t_c)$ est considérée comme symétrique, une borne minimale $m_i$ et une borne maximale $M_i$ peuvent être définies telles que :

$$m_i = \mu_i - \frac{z_i}{2}\sigma_i \text{ et } M_i = \mu_i + \frac{z_i}{2}\sigma_i$$

où $\mu_i$ et $\sigma_i$ sont respectivement la moyenne et l'écart type de la distribution des données d'entrée $X_{i,rest}(t_c)$ correspondant à des mesures effectuées, au repos, lors de la phase de calibration. Le paramètre $z_i$ est une amplitude standardisée. L'amplitude $\Delta_i$ de l'intervalle de repos $R_i$ est égale au produit $\sigma_i z_i$. Ainsi, le paramètre $z_i$ conditionne l'intervalle de repos $R_i$, la valeur de $\sigma_i$ étant obtenue expérimentalement, à partir des données d'entrée $X_{i,rest}(t_c)$. La valeur de $z_i$ permet de déterminer l'intervalle de repos $R_i$ ainsi que son amplitude $\Delta_i$ telle que $\Delta_i = M_i - m_i$.

Sur les figures 3B et 3C, on a illustré la distribution $E_i$ formée par les valeurs $A_i(X_{i,rest}(t_c))$ normalisées (axe des ordonnées de droite). Cette distribution est bornée par $A_i(m_i)$ et par $A_i(M_i)$, correspondant respectivement aux images respectives de $m_i$ et $M_i$ par la fonction de normalisation $A_i$. Ces bornes délimitent un intervalle de référence $A_i(R_i)$, ce dernier correspondant à l'image, par la fonction $A_i$, de l'intervalle de repos $R_i$.

Une deuxième propriété de la fonction de normalisation $A_i$ est que l'image, par cette dernière, des données d'entrée $X_i(t_c)$, résultant de la phase de calibration, et distantes de l'intervalle de repos $R_i$ d'une distance $d_i$ supérieure ou égale à $\alpha_i \Delta_i$, forment des antécédents d'un état de stress $Sl_{i,stress}$, après l'application de la fonction de répartition $S_i$. La valeur $\alpha_i \Delta_i$ est une distance seuil $d_i$. $\alpha_i$ est un paramètre positif lorsque la variation des valeurs de la caractéristique par rapport au stress est positive (cf. figure 3B). $\alpha_i$ est un paramètre négatif lorsque la variation des valeurs de la caractéristique par rapport au stress est négative (cf. figure 3C).

Ainsi :

$$X_i(t_c) > M_i + \alpha_i\Delta_i \Leftrightarrow S_i\left(A_i\left(X_i(t_c)\right)\right) \cong Sl_{i,stress} \quad (14).$$

En considérant une variation positive par rapport au stress, et une valeur $Sl_{i,stress}$ égale à 1, l'expression (14) devient :

$$X_i(t_c) > M_i + \alpha_i\Delta_i \Leftrightarrow S_i\left(A_i\left(X_i(t_c)\right)\right) > 1 - \varepsilon \quad (15)$$

La valeur de $\varepsilon$ est faible devant 1. Elle peut être par exemple égale à 0.1.

Sur les figures 3B et 3C, on a illustré la distribution $F_i$ formée par les valeurs $A_i(X_{i,stress}(t_c))$ normalisées. Sur la figure 3B ($\alpha_i > 0$), la distribution $F_i$ est bornée par une borne dont la valeur est $A_i(M_i + \alpha_i\Delta_i)$, ce qui correspond à l'image, par la fonction $A_i$, de $M_i + \alpha_i\Delta_i$. Sur la figure 3C ($\alpha_i < 0$), la distribution $F_i$ est bornée par une borne dont la valeur est $A_i(m_i + \alpha_i\Delta_i)$, ce qui correspond à l'image, par la fonction $A_i$, de $m_i + \alpha_i\Delta_i$.

La fonction de répartition $S_i$ est par exemple de type sigmoïde, telle que définie en lien avec les expressions (10) et (11). A partir des expressions précédentes, on déduit que :

$$A_i(M_i) = S_i^{-1}(\varepsilon) = ln\left(\frac{\varepsilon}{1-\varepsilon}\right) \quad (16)$$

et

$$A_i(M_i) + \alpha_i[A_i(M_i) - A_i(m_i)] = S_i^{-1}(1-\varepsilon) = S_i^{-1}(\varepsilon) = -A_i(M_i) \quad (17)$$

En utilisant l'hypothèse selon laquelle $A_i$ est une transformée affine (cf. expression (12')), on obtient :

$$a_i = \frac{2ln\left(\frac{1-\varepsilon}{\varepsilon}\right)}{\alpha_i \, (M_i - m_i)} \quad (18)$$

et

$$b_i = ln\left(\frac{\varepsilon}{1-\varepsilon}\right)\left[1 + 2\frac{M_i}{\alpha_i \, (M_i - m_i)}\right] \quad (19)$$

Lorsque la variation de la caractéristique physiologique est négative entre l'état de repos et l'état de stress, comme représenté sur la figure 3C, alors $\alpha_i < 0$, on peut écrire :

$$A_i(m_i) = S_i^{-1}(\varepsilon) = ln\left(\frac{\varepsilon}{1-\varepsilon}\right) \quad (20)$$

et

$$A_i(m_i) + \alpha_i[A_i(M_i) - A_i(m_i)] = S_i^{-1}(1-\varepsilon) = S_i^{-1}(\varepsilon) = -A_i(m_i) \quad (21)$$

Il vient alors :

$$a_i = \frac{2ln\left(\frac{1-\varepsilon}{\varepsilon}\right)}{\alpha_i \, (M_i - m_i)} \quad (22)$$

et

$$b_i = ln\left(\frac{\varepsilon}{1-\varepsilon}\right)\left[1 + 2\frac{m_i}{\alpha_i \, (M_i - m_i)}\right] \quad (23)$$

La fonction de normalisation $A_i$ est conditionnée par le paramètre $\alpha_i$ ainsi que le paramètre $z_i$.

Le paramètre $z_i$ est représentatif de l'intervalle de repos $R_i$, dont l'image, par la fonction de répartition $S_i$ composée avec la fonction de normalisation $A_i$ correspond au niveau de repos $SI_{i,rest}$. Plus précisément, $z_i$ est représentatif de la largeur de l'intervalle de repos. Le paramètre $\alpha_i$ dimensionne la distance seuil $d_i$, entre l'intervalle de repos et un intervalle de stress, dont l'image, par la fonction de répartition $S_i$ composée avec la fonction de normalisation $A_i$, est égale à $SI_{i,stres}$, au terme d'incertitude $\varepsilon$ près.

L'optimisation des paramètres $z_i$ et $\alpha_i$ est effectuée successivement, en commençant par l'optimisation du paramètre $z_i$. Elle est mise en œuvre en utilisant le fait que lors de la phase de calibration, à chaque période de calibration $t_c$, chaque état $SI_i(t_c)$ de l'individu de test est connu. L'optimisation consiste à fixer un paramètre (par exemple $z_i$), et de faire varier l'autre paramètre (par exemple $\alpha_i$) en analysant la performance de classification donnée par la fonction de répartition $f_i((X_i(t_c)) = S_i \circ A_i(X_i(t_c)))$. La fonction de normalisation $A_i$ est paramétrée par les paramètres $a_i$ et $b_i$ tels que définis par les expressions (22) et (23), ces derniers étant eux-mêmes conditionnés par les paramètres $\alpha_i$ et $z_i$, ces derniers ayant une fonction d'hyper-paramètre. La figure 3D illustre la structure hiérarchique de dépendance entre $z_i$ et $\alpha_i$ d'une part et $a_i$ et $b_i$ d'autre part. L'optimisation des paramètres $z_i$ et $\alpha_i$ permet d'aboutir à des paramètres optimisés $z_{i,opt}$ et $\alpha_{i,opt}$. Ces derniers sont utilisés pour définir les paramètres $a_i$ et $b_i$ de la fonction de normalisation $A_i$.

Par performance de classification, il est par exemple entendu la sensibilité, qui correspond à 1 - le taux de faux négatifs, et la spécificité, qui correspond 1 moins le taux de faux positifs. On peut également mettre en œuvre d'autres indicateurs statistiques, par exemple un test de type "d de Cohen", connu de l'homme du métier. Le d de Cohen correspond à une distance inter-classes, en l'occurrence une distance entre les distributions des valeurs respectivement considérées comme représentatives d'un état de stress et d'un état de non stress. L'état de non stress est un état de repos ou un état intermédiaire.

Par exemple, si on dispose de deux distributions statistiques, de valeur moyenne, d'écart-type et d'effectifs

respectivement notés $\mu_1$, $\sigma_1$, $n_1$ et $\mu_2$, $\sigma_2$ et $n_2$, le d de Cohen peut être tel que :

$$d = \frac{\mu_1 - \mu_2}{\sqrt{\dfrac{(\mu_1 - 1)s_1^2 + (\mu_2 - 1)s_2^2}{n_1 + n_2 - 2}}}$$

Les inventeurs ont établi un indicateur de performance de classification c, tel que :

$c = \rho d$ où :

- $\rho$ est un rapport de vraisemblance, tel que $\rho = \dfrac{1-\beta}{\gamma}$ où $\beta$ et $\gamma$ désignent respectivement la sensibilité et la spécificité ;
- $d$ correspond au d de Cohen, précédemment évoqué.

Afin de déterminer le paramètre $z_i$ optimal en termes de performance de classification, le paramètre $\alpha_i$ est fixé à une valeur absolue égale à 0.01. On fait ensuite varier $z_i$ par incréments de 0.25, entre deux bornes prédéterminées, par exemple entre 1 et 5. La valeur optimale de $z_i$, notée $z_{i,opt}$, est telle que :

$$z_{i,opt} = \underset{z_i}{\operatorname{argmax}}(c_{\alpha_i \approx 0}) \quad (25)$$

La notation $c_{\alpha_i \approx 0}$ correspond au fait que l'indicateur de performance est calculé en considérant $\alpha_i$ proche de 0, par exemple égal à 0.01 (lorsque $\alpha_i > 0$) ou -0.01 (lorsque $\alpha_i < 0$). Lors de la détermination de $z_{i,opt}$, les performances de classification sont déterminées en se basant sur deux classes : l'état de repos et l'état de "non repos", ce dernier regroupant les états intermédiaires et l'état de stress. Il s'agit d'évaluer la capacité de la fonction de répartition à classifier correctement l'état de repos.

Après que $z_{i,opt}$ a été déterminé, le paramètre $\alpha_i$ optimal est déterminé fixant le paramètre $z_i$ de telle sorte que $z_i = z_{i,opt}$. On fait ensuite varier $\alpha_i$ par incréments de 0.1, entre deux bornes prédéterminées, par exemple entre 0 et 3 lorsque $\alpha_i > 0$ ou entre -3 et 0 lorsque $\alpha_i < 0$. La valeur optimale de $\alpha_i$, notée $\alpha_{i,opt}$, est telle que :

$$\alpha_{i,opt} = \underset{\alpha_i}{\operatorname{argmax}}\left(c_{z_i = z_{i,opt}}\right) \quad (26)$$

La notation $c_{z_i = z_{i,opt}}$ correspond au fait que l'indicateur de performance est calculé en considérant $z_i = z_{i,opt}$. Lors de la détermination de $\alpha_{i,opt}$, les performances de classification sont déterminées en se basant sur deux classes : l'état de stress et l'état de "non stress", ce dernier regroupant les états intermédiaires et l'état de repos. Il s'agit d'évaluer la capacité de la fonction de répartition à classifier correctement l'état de stress.

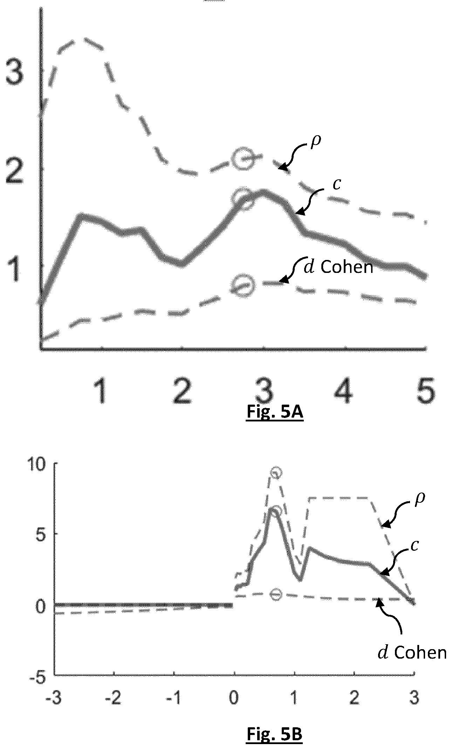

La figure 5A illustre une évolution, en fonction de $z_i$ (axe des abscisses) du rapport de vraisemblance $\rho$, du d de Cohen et de l'indicateur c de performance de classification. Dans cet exemple, la caractéristique étudiée était la variance de la fréquence cardiaque. La figure 5B illustre une évolution, en fonction de $\alpha_i$ (axe des abscisses) du rapport de vraisemblance $\rho$, du d de Cohen et de l'indicateur $c$ de performance de classification. Dans cet exemple, la caractéristique étudiée était également la variance de la fréquence cardiaque. Les figures 5A et 5B illustrent la possibilité de déterminer respectivement $z_{i,opt}$ et $\alpha_{i,opt}$.

Selon une variante, les paramètres $\alpha_{i,opt}$ et $z_{i,opt}$ peuvent être déterminés simultanément. Selon une autre variante, $\alpha_{i,opt}$ peut être déterminé avant $z_{i,opt}$.

La détermination de $z_{i,opt}$ et $\alpha_{i,opt}$ décrite ci-dessus correspond à un exemple, basé sur une détermination de la spécificité, la sensibilité et le d de Cohen. D'une façon plus générale, les paramètres $z_{i,opt}$ et $\alpha_{i,opt}$ peuvent être déterminés sur la base de performances de classification déterminées au cours d'une phase d'apprentissage, au cours de laquelle l'état de chaque individu test est connu.

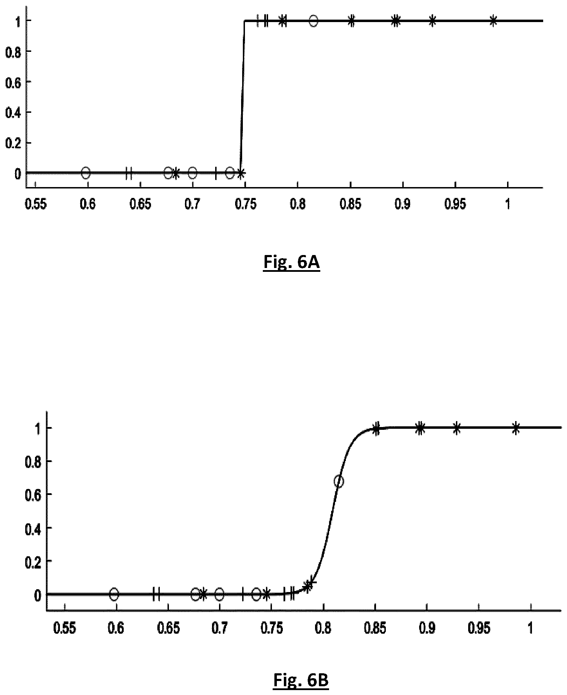

La figure 6A une fonction de répartition $S_i$ composée avec une fonction de normalisation $A_i$ établie avec $z_i = z_{i,opt}$ et $\alpha_i = 0$. La figure 6B une fonction de répartition $S_i$ composée avec une fonction de normalisation $A_i$ établie avec $z_i = z_{i,opt}$ et $\alpha_i = \alpha_{i,opt}$. Sur les figures 6A et 6B, les caractéristiques établies alors que l'individu de test est dans un état de repos, de stress et un état intermédiaire sont respectivement identifiées par des cercles O, des croix + et des étoiles *. Sur la figure 6A, on remarque des erreurs de classification, par exemple des faux positifs : cercles O dont l'ordonnée correspond à un niveau de stress $SI_i$ correspondant à l'état de stress. Cette courbe montre également des faux négatifs :

étoiles * dont l'ordonnée correspond à un niveau de stress $SI_i$ correspondant à l'état de repos. La figure 6B comporte moins d'erreurs de classification.

A la suite de l'étape 130, on dispose de la fonction de normalisation $A_i$, correspondant à la caractéristique physiologique $x_i$.

Etape 140 : Détermination de la fonction d'appartenance $f_i$

Au cours de l'étape 140, la fonction d'appartenance est déterminée selon les expressions (4) ou (5) en utilisant :

- l'éventuelle fonction de standardisation $T_i$ résultant de l'étape 120 ;
- la fonction de répartition $S_i$ prise en compte au cours de l'étape 130 ;
- la fonction de normalisation $A_i$ définie suite à l'étape 130.

Les étapes 100 à 140 peuvent être mises en œuvre respectivement pour différentes caractéristiques physiologiques $x_i$. On obtient autant de fonction de répartition que de caractéristiques physiologiques considérées. Comme précédemment indiqué, les fonctions de répartition peuvent être combinées, en particulier sous la forme d'une somme pondérée, de façon à établir un niveau de stress.

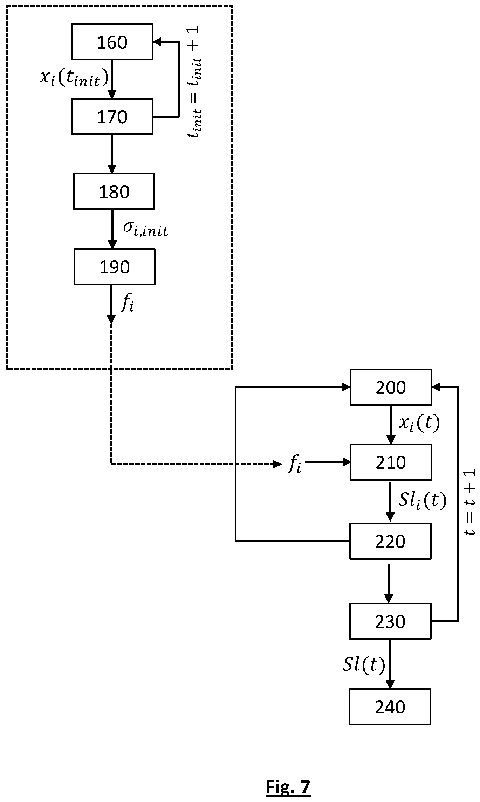

La figure 7 représente les principales étapes de mise en œuvre d'un procédé de détermination d'un état de stress, en mettant en œuvre une fonction d'appartenance telle que déterminée suite à l'étape 140.

Au cours de l'étape 200, on effectue une mesure d'une, ou de plusieurs caractéristique physiologique $x_i(t)$ à une période de mesure $t$. Chaque caractéristique physiologique $x_i$ mesurée à chaque période de mesure correspond à une caractéristique physiologique adressée lors de la calibration.

Au cours de l'étape 210, on applique la fonction d'appartenance $f_i$, préalablement définie pour chaque caractéristique physiologique, à la valeur $x_i(t)$ de la caractéristique physiologique à la période de mesure, de façon à déterminer un niveau de stress $SI_i(t) = f_i(x_i(t))$ relativement à la caractéristique physiologique $x_i$.

Au cours de l'étape 220, on détermine le niveau de stress de l'utilisateur $SI(t)$, durant la période de mesure $t$, en fonction de chaque niveau de stress $SI_i(t)$ établi, pour chaque caractéristique physiologique adressée, durant l'étape 210.

Au cours de l'étape 230, le procédé est réitéré pour une autre période de mesure ou une sortie d'algorithme est décidée.

Selon une variante, faisant l'objet des étapes 160 à 190, le procédé comporte une phase d'initialisation, au cours de laquelle l'utilisateur est exclusivement dans un état de repos, ou considéré dans un état de repos. L'initialisation consiste à effectuer des mesures d'au moins une caractéristique physiologique $x_i(t_{init})$, de façon à estimer une plage de variation. Les mesures sont mises en œuvre au cours de différentes périodes d'initialisation $t_{init}$. Un indicateur de dispersion est déterminé, caractérisant la plage de variation de la caractéristique $x_i$ durant les différentes périodes d'initialisation $t_{init}$. L'indicateur de dispersion peut par exemple être un écart type $\sigma_{i,init}$.

L'indicateur de dispersion $\sigma_{i,init}$ ainsi déterminé est utilisé pour déterminer les bornes $M_i$ et $m_i$ de l'intervalle de repos $R_i$ à partir de $z_i$. On utilise alors les expressions :

$$m_i = \mu_i - \frac{z_{i,opt}}{2}\sigma_{i,init} \quad (27)$$

et

$$M_i = \mu_i + \frac{z_{i,opt}}{2}\sigma_{i,init} \quad (28)$$

Ainsi, selon cette variante, l'initialisation n'est effectuée uniquement avec des valeurs de la caractéristique mesurées lors de la calibration, alors que l'utilisateur est considéré comme étant dans un état de repos. L'initialisation ne nécessite pas d'effectuer des mesures d'une caractéristique physiologique alors que l'utilisateur se trouve dans un état de stress. Un avantage de la méthode est que l'initialisation est plus rapide et plus simple à réaliser. Un autre avantage est que l'initialisation peut être renouvelée périodiquement, afin de tenir compte d'une éventuelle variabilité physiologique de l'utilisateur. L'initialisation étant particulièrement simple à réaliser, et son renouvellement peut fréquemment peut être effectué fréquemment.

Les étapes 160 à 190 sont alors les suivantes :

Etape 160 : réalisation d'une mesure d'initialisation $x_i(t_{init})$

Etape 170 : réitération de l'étape 160 ou passage à l'étape 180

Etape 180 : calcul de l'indicateur de dispersion $\sigma_{i,init}$.

Etape 190 : utilisation de l'indicateur de dispersion $\sigma_{i,init}$ pour ajuster la fonction d'appartenance $f_i$ utilisée lors de l'étape 210, en combinant les expressions (27) et (28) avec les expressions (18) et (19) ou (22) et (23).

Selon une autre variante, l'initialisation n'est pas effectuée et la fonction d'appartenance, relativement à une caractéristique, voire à chaque caractéristique considérée, est établie sans ajustement de la fonction d'appartenance résultant de la calibration.

L'invention pourra être mise en œuvre pour le suivi du niveau de stress d'utilisateurs. Il peut par exemple s'agir d'un suivi du niveau de stress en milieu professionnel, ou du suivi du niveau de stress chez des utilisateurs sujets à une anxiété lors de situations particulières, par exemple dans un moyen de transport. Elle peut également être appliquée au suivi du niveau de stress d'un sportif. Ainsi, l'état de stress peut être un état d'anxiété ou d'effort, qu'il s'agisse d'effort physique ou mental, ou un état d'excitation. L'état de stress peut correspondre à une charge mentale de l'utilisateur : joie, colère, angoisse, fatigue, sensation de manque. L'invention peut également s'appliquer à la détermination d'un état de veille ou d'endormissement.

**Revendications**

1. Procédé de détermination d'une fonction d'appartenance ($f_i$), la fonction d'appartenance permettant d'estimer un niveau de stress ($SI_i$) d'un utilisateur à partir d'une caractéristique physiologique ($x_i$) mesurée sur l'utilisateur, durant une période de mesure ($t$), la fonction d'appartenance variant, en fonction de la caractéristique physiologique, entre :

   • une première valeur ($SI_{i,rest}$), représentative d'un état de repos ;
   • et une deuxième valeur ($SI_{i,stress}$), représentative d'un état de stress ;

   la fonction d'appartenance ($f_i$) prenant en compte une fonction de répartition ($S_i$), préalablement définie, la fonction de répartition étant une fonction continue et monotone, la fonction de répartition ($S_i$) étant appliquée à une caractéristique normalisée ($A_i(x_i(t), A_i(T_i(x_i(t))))$), établie à partir d'une caractéristique physiologique mesurée ($x_i(t)$), le procédé comportant les étapes suivantes :

   - a) mesures de valeurs ($x_i(t_c)$) d'une caractéristique physiologique ($x_i$), en différentes périodes de calibration ($t_c$), sur au moins un individu de test, chaque individu de test étant placé, en chaque période de calibration, soit dans un état de repos, soit dans un état de stress, soit dans un état intermédiaire, l'état intermédiaire étant un état dans lequel l'individu de test est considéré comme n'étant ni dans un état de stress, ni dans un état de repos;
   - b) éventuellement, application d'une fonction de standardisation ($T_i$) aux valeurs mesurées au repos, lors de l'étape a), pour obtenir des paramètres standardisés, la fonction de standardisation ($T_i$) étant concave;
   - c) à partir :

      • des valeurs mesurées lors de l'étape a) et/ou éventuellement standardisées lors de l'étape b) ;
      • de la fonction de répartition ($S_i$) ;

   détermination d'une fonction de normalisation ($A_i$), la fonction de normalisation étant destinée à être appliquée aux valeurs mesurées de la caractéristique physiologique, éventuellement standardisées, afin d'établir des valeurs normalisées, la détermination de la fonction de normalisation comportant :

      • une optimisation d'un intervalle de repos ($R_i$), représentatif des valeurs de la caractéristiques mesurées lorsque chaque individu de test est au repos ;
      • une optimisation d'une distance seuil ($d_i$), entre l'intervalle de repos, et un intervalle de stress, représentatif des valeurs de la caractéristique mesurées lorsque chaque individu de test est dans un état de stress;

   - d) détermination de la fonction d'appartenance ($f_i$) associée à la caractéristique physiologique, la fonction d'appartenance correspondant à une composition de la fonction de répartition ($S_i$), de la fonction de normalisation ($A_i$) et de l'éventuelle fonction de standardisation ($T_i$).

2. Procédé selon la revendication 1, dans lequel lors de l'étape a), les valeurs de la caractéristique physiologique mesurées alors que chaque individu de test est dans un état de stress, de repos ou intermédiaire sont regroupées respectivement une classe de stress, une classe de repos et une classe intermédiaire, et dans lequel lors de l'étape c),

    • l'intervalle de repos ($R_i$) est optimisé de façon à optimiser une classification, par la fonction de répartition ($S_i$), de valeurs normalisées ($A_i(x_i(t_c)),A_i(T_i(x_i(t_c))$), correspondant respectivement à la classe de repos et à une union de la classe intermédiaire et de la classe de stress;
    • la distance seuil ($d_i$) est optimisée de façon à optimiser une classification, par la fonction de répartition ($S_i$), de valeurs normalisées ($A_i(x_i(t_c)),A_i(T_i(x_i(t_c))$) correspondant respectivement à la classe de stress et une union de la classe intermédiaire et de la classe de repos.

3. Procédé selon la revendication 2, dans lequel l'étape c), comporte une détermination :

    • d'un indicateur de classification des valeurs normalisées entre la classe de repos et une union de la classe intermédiaire et de la classe de stress, l'indicateur de classification étant basé sur une proportion de faux positifs et de faux négatifs ;
    • d'un indicateur de classification des valeurs normalisées entre la classe de stress et une union de la classe intermédiaire et de la classe de repos, l'indicateur de classification étant basé sur une proportion de faux positifs et de faux négatifs.

4. Procédé selon l'une quelconque des revendications précédentes, dans lequel l'étape c) comporte :

    • une détermination d'un intervalle de repos optimal ($R_i$), en fixant la distance seuil ($d_i$);
    • puis une détermination de la distance seuil optimale ($d_i$), en fonction de l'intervalle de repos optimal ($R_i$).

5. Procédé selon l'une quelconque des revendications précédentes, dans lequel l'étape c) comporte une optimisation d'une étendue ($z_i$) de l'intervalle de repos ($R_i$).

6. Procédé selon la revendication 5, dans lequel l'étape c) comporte une optimisation d'un facteur d'échelle ($\alpha_i$), la distance seuil ($d_i$) étant déterminée en multipliant le facteur d'échelle ($\alpha_i$) par l'étendue de l'intervalle de repos ($z_i$).

7. Procédé selon l'une quelconque des revendications précédentes, dans lequel la fonction de normalisation ($A_i$) est une fonction affine, l'étape c) comportant une détermination des paramètres ($a_i,b_i$) de la fonction affine à partir de l'intervalle de repos ($R_i$) et de la distance seuil ($d_i$) optimisés.

8. Procédé selon l'une quelconque des revendications précédentes, dans lequel la fonction de standardisation ($T_i$) est de type logarithmique ou racine carrée.

9. Procédé selon l'une quelconque des revendications précédentes, comportant :

    • suite à l'étape a), calcul d'un coefficient d'asymétrie ($k_i$) d'une distribution ($D_i$) des valeurs de la caractéristique physiologique mesurées lorsque chaque individu de test sont au repos, le coefficient d'asymétrie quantifiant l'asymétrie de la distribution ;
    • comparaison du coefficient d'asymétrie par rapport à un seuil prédéterminé ($k_{th}$);
    • en fonction de la comparaison, mise en œuvre de l'étape b).

10. Procédé selon l'une quelconque des revendications précédentes, dans lequel :

    • les étapes a) à d) forment un enchainement d'étapes associé à la caractéristique physiologique ($x_i$) mesurée lors de l'étape a), et conduisant à la détermination d'une fonction d'appartenance ($f_i$) associée à la caractéristique physiologique ;
    • le procédé comporte différents enchainements d'étapes a) à d), respectivement associés à différentes caractéristiques physiologiques ($x_i$), de façon à déterminer différentes fonctions d'appartenance ($f_i$), chaque fonction d'appartenance étant respectivement associée à une caractéristique physiologique.

11. Procédé de détermination d'un niveau de stress ($Sl_i$) d'un utilisateur, en fonction d'une caractéristique physiologique ($x_i$) mesurée sur l'utilisateur, durant une période de mesure (t), dont la valeur est susceptible de varier en fonction du niveau de stress de l'utilisateur, le procédé comportant les étapes suivantes :

- i) mesure d'une valeur de la caractéristique physiologique ($x_i(t)$) durant une période de mesure ($t$);
- ii) application d'une fonction d'appartenance ($f_i$) à la valeur de la caractéristique physiologique ($x_i(t)$) mesurée lors de l'étape i) ;
- iii) estimation du niveau de stress ($SI_t(t)$) de l'utilisateur en fonction de la valeur de la fonction d'appartenance calculée lors de l'étape ii) ;

le procédé étant **caractérisé en ce que** la fonction d'appartenance ($f_i$) est une fonction déterminée selon un procédé objet de l'une quelconque des revendications précédentes.

12. Procédé de détermination d'un niveau de stress d'un utilisateur, en fonction d'une pluralité de caractéristiques physiologiques de l'utilisateur, la valeur de chaque caractéristique physiologique étant susceptible de varier en fonction du niveau de stress de l'utilisateur, le procédé comportant les étapes suivantes :

- i) mesure de valeurs de différentes caractéristiques physiologiques ($x_1 \ldots x_l$) durant une période de mesure ($t$);
- ii) pour chaque valeur de caractéristique physiologique mesurée ($x_i(t)$) lors de l'étape i), application d'une fonction d'appartenance ($f_1 \ldots f_l$) associée à la caractéristique physiologique
- iii) estimation du niveau de stress ($SI$) en combinant les valeurs des fonctions d'appartenance calculées lors de l'étape i) ;
- le procédé étant **caractérisé en ce qu'**au moins une fonction d'appartenance, associée à un caractéristique physiologique, est une fonction déterminée selon un procédé objet de l'une quelconque des revendications 1 à 10.

13. Procédé selon l'une quelconque des revendications 11 ou 12 comportant une phase d'initialisation, regroupant différentes périodes d'initialisation ($t_{init}$), au cours desquelles l'utilisateur est considéré comme étant dans un état de repos, la phase d'initialisation comportant :

- une mesure, en chaque période d'initialisation, d'une caractéristique physiologique ($x_i(t_{init})$);
- une détermination d'un indicateur de dispersion ($\sigma_{init}$) des valeurs mesurées à chaque période d'initialisation ;

le procédé comportant, préalablement aux étapes i) à iii), un ajustement de la fonction d'appartenance ($f_i$), établie pour la caractéristique physiologique, en prenant en compte l'indicateur de dispersion.

14. Dispositif (1) destiné à mesurer un niveau de stress d'un utilisateur, le dispositif comportant :

- un capteur (2), destiné à être placé contre le corps de l'utilisateur, le capteur étant configuré pour mesurer une caractéristique physiologique ($x(t)$) de l'utilisateur ;
- un microprocesseur (4), configuré pour recevoir une mesure du capteur, le microprocesseur étant programmé pour mettre en œuvre le procédé selon la revendication 11 ou la revendication 12.

**Fig. 1**

**Fig. 2**

**Fig. 3A**

**Fig. 3B**

Fig. 3C

Fig. 3D

**Fig. 4A**

**Fig. 4B**

**Fig. 4C**

**Fig. 5A**

**Fig. 5B**

**Fig. 6A**

**Fig. 6B**

**Fig. 7**

**EP 3 841 964 A1**

Europäisches Patentamt
European Patent Office
Office européen des brevets

# RAPPORT DE RECHERCHE EUROPEENNE

Numéro de la demande

EP 20 21 7202

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X | CHARBONNIER S ET AL: "A Multi-feature Fuzzy Index to Assess Stress Level from Bio-signals", 2018 40TH ANNUAL INTERNATIONAL CONFERENCE OF THE IEEE ENGINEERING IN MEDICINE AND BIOLOGY SOCIETY (EMBC), IEEE, 18 juillet 2018 (2018-07-18), pages 1086-1089, XP033431946, DOI: 10.1109/EMBC.2018.8512499 | 1-10 | INV. A61B5/00 A61B5/16 G06N7/02 |
| A | * sections I-III * ----- | 11-13 | |
| A | EP 3 305 186 A1 (COMMISSARIAT ENERGIE ATOMIQUE [FR]) 11 avril 2018 (2018-04-11) * abrégé * ----- | 1-13 | |

| DOMAINES TECHNIQUES RECHERCHES (IPC) |
|---|
| A61B G06N G06K G06F G16H |

Le présent rapport a été établi pour toutes les revendications

| Lieu de la recherche | Date d'achèvement de la recherche | Examinateur |
|---|---|---|
| Berlin | 14 mai 2021 | Domingo Vecchioni, M |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

## ANNEXE AU RAPPORT DE RECHERCHE EUROPEENNE
## RELATIF A LA DEMANDE DE BREVET EUROPEEN NO.

EP 20 21 7202

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

14-05-2021

| Document brevet cité au rapport de recherche | | Date de publication | Membre(s) de la famille de brevet(s) | | Date de publication |
|---|---|---|---|---|---|
| EP 3305186 | A1 | 11-04-2018 | EP | 3305186 A1 | 11-04-2018 |
| | | | FR | 3057152 A1 | 13-04-2018 |

Pour tout renseignement concernant cette annexe : voir Journal Officiel de l'Office européen des brevets, No.12/82

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- FR 1856504

**Littérature non-brevet citée dans la description**

- A comparison of wearable and stationary sensors for stress detection. *2016 IEEE international conférence on Systems, Man and Cybernetics (SMC),* Octobre 2016
- **KUMAR M.** Fuzzy techniques for subjective workload-score modeling under uncertainties. *IEEE transactions on systems, man and cybernetics- part B,* Décembre 2008, vol. 38 (6
- **SANTOS SIERRA A. et al.** Real-time stress détection by means of physiological signais. *Recent Application in Biometrics,* 27 Juillet 2011
- **CHARBONNIER, SYLVIE et al.** A Multi-feature Fuzzy Index to Assess Stress Level from Bio-signals. Conférence proceedings : Annual International Conférence of the IEEE Engineering in Medicine and Biology Society. *IEEE Engineering in Medicine and Biology Society. Conference,* 2018, 1086-1089